# EUROPEAN PATENT APPLICATION

(11) **EP 4 835 902 A1**
(43) Date of publication of application: **04.11.2026**
(21) Application number: 25173776.3
(22) Date of filing: 30.04.2025
(51) Int. Cl.: G16H 20/13, G16H 40/67

(54) **LIFE CROWN SMART EMERGENCY PILLBOX SYSTEM**

(71) Applicant: RHAIGroup, 4000 Roskilde, Sjælland (DK)
(72) Inventor: Hussain, Rizan, 4000 Roskilde (DK)

(57) **Abstract**

The "LifeCrown" system offers a comprehensive and intelligent home healthcare solution, integrating medical management, emergency response, and psychological support into one seamless experience. Designed primarily for elderly individuals and patients with chronic conditions or mobility impairments, the system is composed of two interconnected units: a lightweight drawer unit for daily medication and a central fixed unit serving as the intelligent core of the system.

The central unit features advanced sensors that monitor vital signs such as blood pressure, heart rate, and movement activity, and automatically unlock the correct medication compartment according to the patient's treatment plan. In case of non-response, the system activates a graduated three-level emergency protocol: starting with local alerts, escalating to communication with caregivers, and ultimately contacting emergency services with GPS location data and unlocking the home door for paramedics.

The system provides emotional support through a circular screen that displays an animated, friendly face inspired by social robots, along with voice interaction in natural language. All emergency interactions are recorded securely using an encrypted audio-video module, ensuring privacy and traceability.

A digital medical card embedded in the system securely stores essential health records and transmits them automatically during critical emergencies to medical personnel. The system also features synchronization with healthcare providers to update prescriptions and schedules in real-time, removing the need for manual intervention.

The LifeCrown system runs on standard power with a high-capacity lithium-polymer battery backup, ensuring continuous functionality even during outages. A modern Type-C port provides efficient recharging.

LifeCrown stands as a model for the future of compassionate and intelligent home-based healthcare - merging technology with human touch to ensure safety, dignity, and well-being.

## Description

The LifeCrown system is a next-generation smart healthcare platform designed to protect and support vulnerable patients at home-especially the elderly, individuals with chronic conditions, and those living with physical or cognitive impairments. LifeCrown stands as a fully integrated solution, merging medication management, emergency responsiveness, real-time monitoring, emotional engagement, and seamless medical synchronization.

### System Architecture:

The LifeCrown system is composed of two physically interconnected yet functionally distinct components:
1. A lightweight unit containing seven rows of drawers representing seven days of the week, each row subdivided into five compartments (morning, noon, afternoon, evening, night).
2. A fixed central unit acting as the intelligent hub (the "brain") of the system. It houses the processors, health sensors, communication modules, visual interface, speaker, microphone, and emergency functions.

### Medication Management:

At the core of LifeCrown is an intelligent time-based medication delivery system. Healthcare providers define dosage schedules remotely using medical dashboards connected to the system. The patient's drawers remain locked until the exact scheduled time. If the patient misses the dose, the system activates a first-level alert. If the patient fails to respond after multiple stimuli, LifeCrown analyzes vital signs to determine the appropriate escalation path.

### Health Monitoring and Sensors:

Embedded sensors within the device continuously track patient data including heart rate, blood pressure, motion detection, and responsiveness. The data is processed locally and stored securely. When abnormal patterns are detected, the system reacts autonomously.

### Emergency Escalation Logic:

The system features a three-level emergency escalation protocol:
- Level 1 (Standard Alert): Visual LED blinking and soft auditory alerts designed to attract patient attention.
- Level 2 (Intermediate Alert): Elevated warning sounds, re-evaluation of vital signs, and auto-calling registered caregivers or nurses.
- Level 3 (Critical Emergency): The system transmits the patient's geolocation to emergency responders, initiates audio-visual contact through an encrypted module, and automatically unlocks the front door to allow paramedics safe entry.

### Interactive Interface:

The unit includes a circular display showing a soft human-like animated face modeled after companion robots. This feature plays a dual role-providing visual interaction and emotional comfort. The voice assistant engages the patient using natural speech, offering reminders, instructions, and general encouragement.

### Digital Medical Card:

Each patient is assigned an encrypted digital medical card stored within the system. This card contains essential medical history, ongoing treatments, medication allergies, and critical alerts. In emergencies, this data is securely transmitted to medical teams.

### Power and Security:

The system runs on standard AC power and includes a built-in lithium-polymer backup battery capable of powering the device for over 24 hours in case of an outage. The charging system supports modern USB-C technology for quick and safe recharge cycles. The entire architecture of LifeCrown emphasizes data privacy, with end-to-end encryption used for all communications, including emergency alerts and medical data synchronization.

### Synchronization with Medical Infrastructure:

LifeCrown connects wirelessly to a centralized medical platform that allows real-time updates of treatment plans, medication changes, and alerts. Physicians and healthcare staff can remotely monitor patient status and receive automated reports without requiring patient involvement.

### Design Philosophy and User Experience:

The LifeCrown project was designed with deep consideration for user dignity, simplicity, and visual comfort. The interactive face and audio guidance make the device less intimidating for seniors. The daily drawer unit is lightweight and ergonomic, allowing easy placement on bedside tables or wheelchairs. All functions are autonomous-requiring no smartphone, no screen-touch interaction, and no technological familiarity from the patient.

### Recording, Documentation, and Compliance:

Every emergency is logged through the system's secure internal storage using both video and audio, ensuring full traceability of events. These records can be reviewed by medical professionals or authorized family members to evaluate system responses and patient conditions. LifeCrown is designed in full compliance with European healthcare and safety standards.

### Installation and Setup:

The system is plug-and-play. Upon first use, a quick calibration phase occurs where sensors adjust to the patient's baseline vitals. Medical staff can preprogram the schedule, emergency contacts, and thresholds through a secure web-based platform.

### Future Expandability:

The modular architecture of LifeCrown allows future expansion to include blood glucose monitors, ECG tracking, environmental sensors (e.g., smoke, fall detection), or integration with Al-based health prediction engines.

### Conclusion:

LifeCrown is not just a device-it's a life-preserving, dignity-respecting companion engineered to bring hospital-grade protection and comfort into the home. It represents the forefront of healthcare technology merged with emotional intelligence, setting a new standard for autonomous, personalized, and responsive medical care for those who need it most.

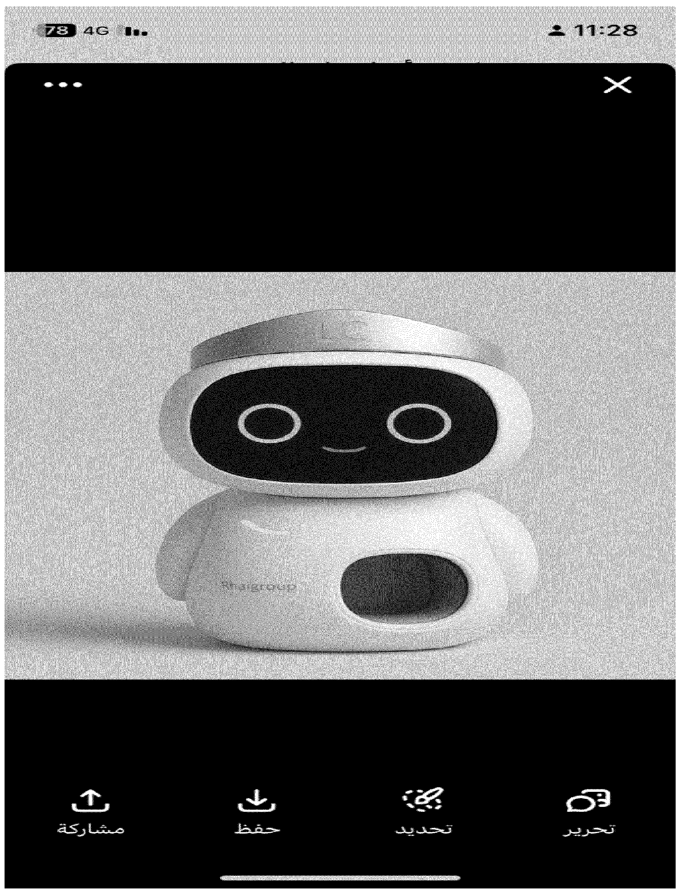

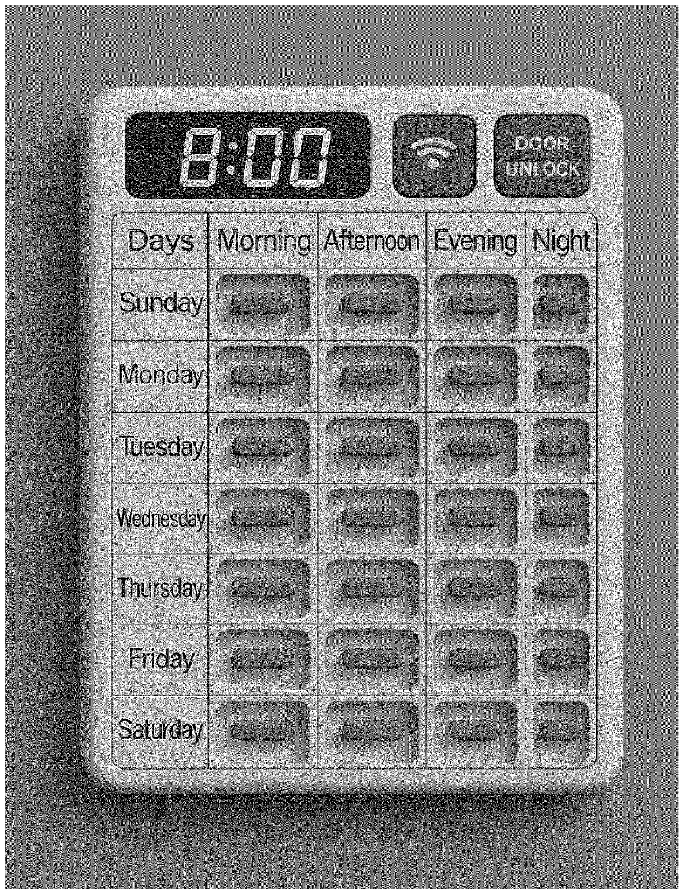

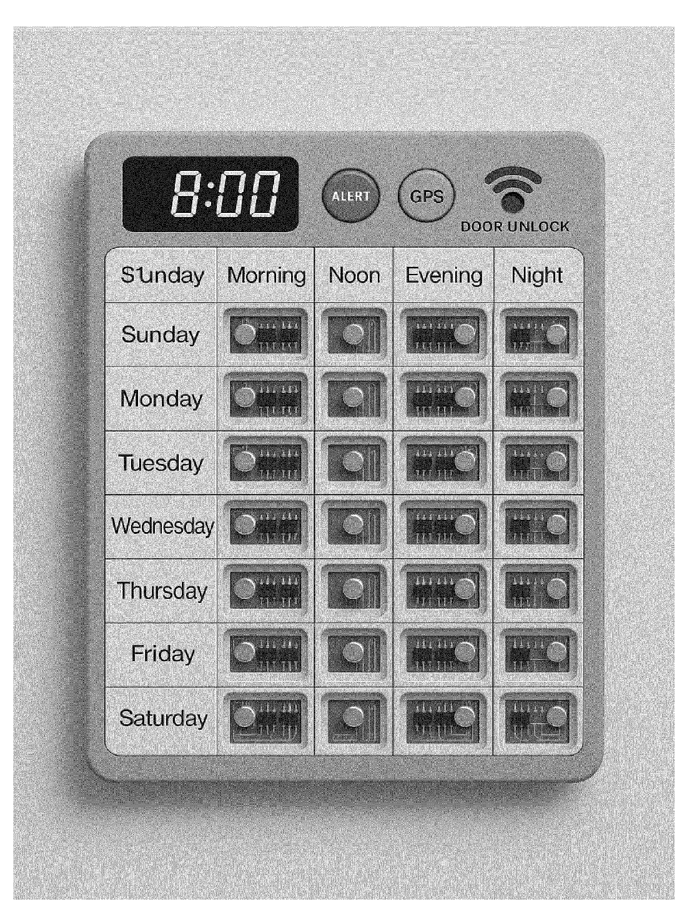

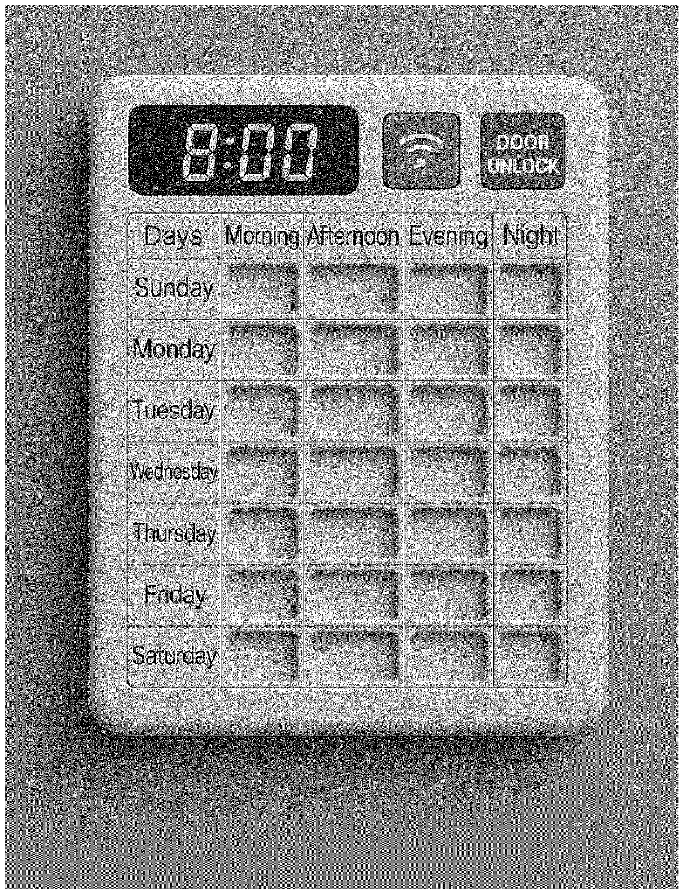

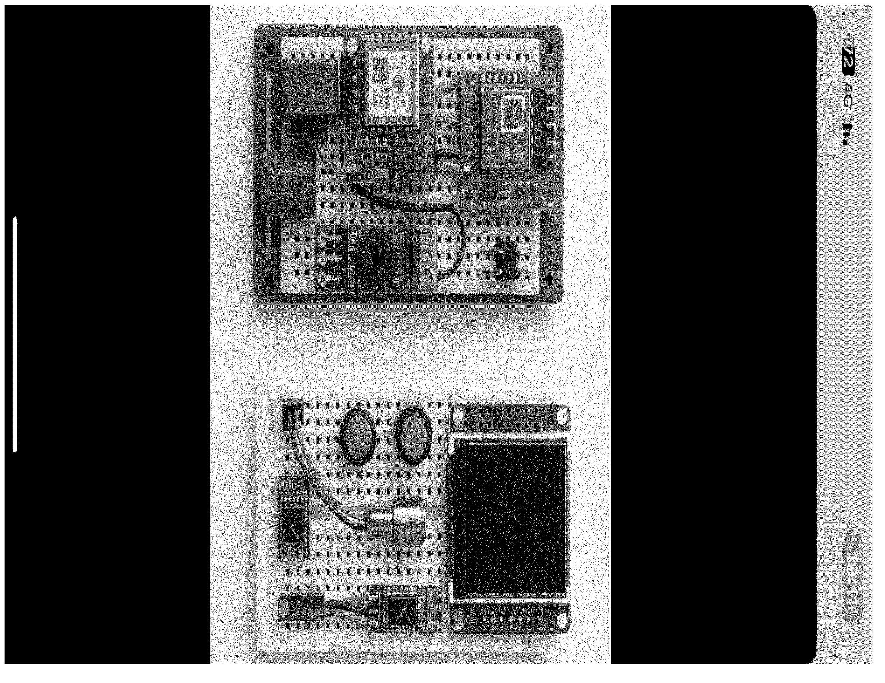

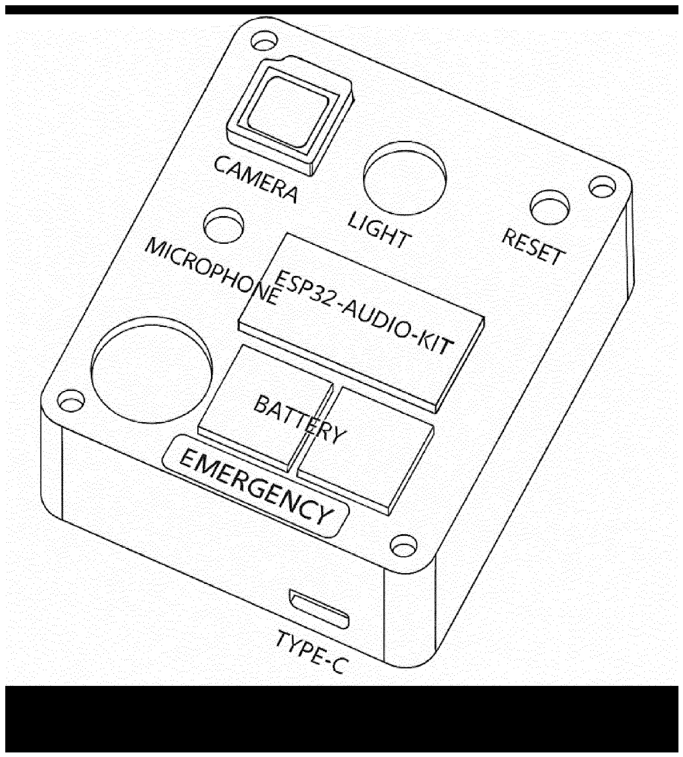

## Claims

1. A dynamic and intelligent system for scheduling daily medication doses over seven days and five time periods per day, allowing automatic remote customization by healthcare providers according to patient health conditions, ensuring correct and timely medication intake without manual intervention.

2. An intelligent electronic locking system that verifies both the time and the patient's health status before allowing any drawer to be opened, preventing unauthorized or incorrect access while ensuring emergency access when needed.

3. A multi-stage alert system that categorizes emergencies into normal (light sound and flash), moderate (escalated sound/flash and nurse contact), and critical (strong alarm activation and direct ambulance notification with patient location).

4. An integrated medical sensing unit capable of continuously monitoring vital signs such as heart rate, blood pressure, and movement, analyzing data in real-time to detect critical health risks like unconsciousness or falls, triggering automatic emergency response.

5. An intelligent emergency communication unit that automatically initiates audio-visual contact with nursing staff under moderate emergencies and directly with ambulance teams under critical emergencies, transmitting real-time health data and geolocation.

6. A privacy-protected optical lens cover that only opens automatically during certified emergencies, ensuring complete protection of patient privacy outside of active emergencies.

7. An embedded GPS system that activates automatically during emergencies to transmit precise patient location to emergency responders or healthcare providers.

8. A smart emergency alarm system combining flashing red light, loud audio alarm, and unconsciousness detection with automatic front door unlocking when no response is detected, enabling emergency access.

9. A manual SOS button allowing patients or caregivers to manually trigger full emergency protocols including alarm activation, location transmission, and contacting emergency services in case of unrecognized emergencies.

10. An integrated smart recording system that automatically documents audio-visual data during emergencies while encrypting sensitive information, combined with a digital medical ID containing essential patient data sent to emergency services.

11. An automatic synchronization system that continuously updates treatment plans and medication schedules between the device and healthcare providers without requiring patient intervention, ensuring seamless healthcare continuity.

12. An internal backup power unit utilizing high-efficiency lithium polymer batteries to guarantee uninterrupted device functionality during power outages and allowing rapid recharging via modern USB Type-C connectors.

13. A remote control system that allows authorized medical personnel to update medication schedules, unlock specific drawers, or activate emergency modes remotely with full command tracking for medical transparency.

14. An intelligent voice assistant with natural human interaction designed to guide the patient through medication schedules, provide daily motivational and medical advice, and automatically trigger emergency responses based on real-time health data analysis.

15. An interactive 4-inch circular display showing current time, upcoming medication schedules, emergency status, and live caller ID during emergency communication, enhancing psychological support and emotional stability for the patient.
